(19) Europäisches Patentamt / European Patent Office / Office européen des brevets

(11) **EP 4 748 377 A1**

(12) **EUROPEAN PATENT APPLICATION**
published in accordance with Art. 153(4) EPC

(43) Date of publication:
**27.05.2026 Bulletin 2026/22**

(21) Application number: **24843207.2**

(22) Date of filing: **19.07.2024**

(51) International Patent Classification (IPC):
**A61K 31/7008** (2006.01)    **A61K 9/08** (2006.01)
**A61K 47/10** (2017.01)    **A61K 47/38** (2006.01)
**A61P 31/04** (2006.01)

(52) Cooperative Patent Classification (CPC):
**A61K 9/08; A61K 31/7008; A61K 47/10;
A61K 47/38; A61P 31/04**

(86) International application number:
**PCT/JP2024/026008**

(87) International publication number:
**WO 2025/018419 (23.01.2025 Gazette 2025/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **20.07.2023 JP 2023118582**

(71) Applicant: **SENJU USA, INC.
Torrance, CA 90503 (US)**

(72) Inventor: **NISHIDA, Yumena
Osaka-shi, Osaka 541-0048 (JP)**

(74) Representative: **Mewburn Ellis LLP
Aurora Building
Counterslip
Bristol BS1 6BX (GB)**

(54) **WATER-BASED LIQUID PREPARATION**

(57)    Provided is a formulation technology relating to an aqueous liquid formulation comprising arbekacin and/or a salt thereof. More specifically, provided is an aqueous liquid formulation comprising arbekacin and/or a salt thereof, hydroxypropyl methylcellulose, and propylene glycol.

Fig. 1

**Description**

Technical Field

The present disclosure relates to an aqueous liquid formulation comprising arbekacin and/or a salt thereof, hydroxypropyl methylcellulose, and propylene glycol, as well as its applied technology.

Background Art

Arbekacin is known as a type of antibiotic.

Citation List

Patent Literature

    PTL 1: US9511143B2
    PTL 2: JP7224390B2

Non-patent Literature

NPL 1: Clin Pharmacol. 2014 Sep 26; 6: 139-48.

Summary of Invention

The present inventors found that when hydroxypropyl methylcellulose is added to an aqueous liquid formulation containing arbekacin to increase its viscosity, the viscosity tends to decrease over time. Further examination revealed that the decrease in viscosity can be suppressed by using propylene glycol.

The present disclosure includes, for example, the subject matter described in the following items.

Item 1.

An aqueous liquid formulation comprising arbekacin and/or a salt thereof, hydroxypropyl methylcellulose, and propylene glycol.

Item 2.

The aqueous liquid formulation according to Item 1, wherein the total concentration of arbekacin and/or a salt thereof is about 0.05 w/v% to about 5.0 w/v% in terms of arbekacin.

Item 3.

The aqueous liquid formulation according to Item 1 or 2, wherein the concentration of hydroxypropyl methylcellulose is about 0.2 w/v% to about 3.5 w/v%.

Item 4.

The aqueous liquid formulation according to any one of Items 1 to 3, wherein the concentration of propylene glycol is about 0.1 w/v% to about 10 w/v%.

Item 5.

The aqueous liquid formulation according to any one of Items 1 to 4, wherein the total concentration of arbekacin and/or a salt thereof is about 0.05 w/v% to about 5.0 w/v% in terms of arbekacin, and the concentration of hydroxypropyl methylcellulose is about 0.2 w/v% to about 3.5 w/v%.

Item 6.

The aqueous liquid formulation according to any one of Items 1 to 5, wherein the total concentration of arbekacin and/or a salt thereof is about 0.05 w/v% to about 5.0 w/v% in terms of arbekacin, and the concentration of propylene glycol is about 0.1 w/v% to about 10 w/v%.

Item 7.

The aqueous liquid formulation according to any one of Items 1 to 6, wherein the concentration of hydroxypropyl methylcellulose is about 0.2 w/v% to about 3.5 w/v%, and the concentration of propylene glycol is about 0.1 w/v% to about 10 w/v%.

Item 8.

The aqueous liquid formulation according to any one of Items 1 to 7, wherein the total concentration of arbekacin and/or a salt thereof is about 0.05 w/v% to about 5.0 w/v% in terms of arbekacin, the concentration of hydroxypropyl methylcellulose is about 0.2 w/v% to about 3.5 w/v%, and the concentration of propylene glycol is about 0.1 w/v% to about 10 w/v%.

Item 9.

The aqueous liquid formulation according to any one of Items 1 to 8, wherein the mass ratio of the total content of arbekacin and/or a salt thereof in terms of arbekacin and the content of propylene glycol is 1:about 0.02 to 1:about 200.

Item 10.

The aqueous liquid formulation according to any one of Items 1 to 9, wherein the mass ratio of the content of hydroxypropyl methylcellulose and the content of propylene glycol is 1:about 0.028 to 1:about 50.

Item 11.

The aqueous liquid formulation according to any one of Items 1 to 10, wherein the mass ratio of the total content of arbekacin and/or a salt thereof in terms of arbekacin and the content of hydroxypropyl methylcellulose is 1:about 0.04 to 1:about 70.

Item 12.

The aqueous liquid formulation according to any one of Items 1 to 11, wherein the mass ratio of the total content of arbekacin and/or a salt thereof in terms of arbekacin, the content of hydroxypropyl methylcellulose, and the content of propylene glycol is 1:about 0.04:about 0.02 to 1:about 70:about 200.

Item 13.

The aqueous liquid formulation according to any one of Items 1 to 12, which is an eye drop.

Item 14.

A method for stabilizing the viscosity of an aqueous liquid formulation, comprising preparing an aqueous liquid formulation comprising arbekacin and/or a salt thereof, hydroxypropyl methylcellulose, and propylene glycol.

Brief Description of Drawing

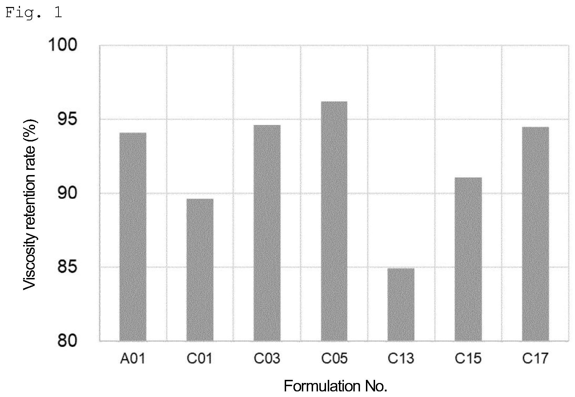

Fig. 1 shows the viscosity retention rate at 50°C after two weeks.

Description of Embodiments

Each embodiment included in the present disclosure is described in more detail below.

## 1. Definition

It should be understood that the terms used in the present specification are used in the sense commonly used in the art, unless otherwise specified. Therefore, unless otherwise defined, all technical and scientific terms used in the present specification have the same meaning as commonly understood by a person skilled in the art to which the present disclosure pertains. In case of conflict, the present specification (including the definition) shall prevail.

In the present specification, the term "about" means $\pm10\%$ of the value that follows, unless otherwise specified.

In the present specification, the "aqueous liquid formulation" is a liquid formulation that contains water as a base.

In the present specification, "arbekacin" is a compound known as an aminoglycoside antibiotic having strong antibacterial activity against methicillin-resistant *Staphylococcus aureus* (MRSA), and refers to 3-amino-3-deoxy-$\alpha$-D-glucopyranosyl-(1-+6)-[2,6-diamino-2,3,4,6-tetradeoxy-$\alpha$-D-erythro-hexopyranosyl-(1$\rightarrow$4)]-1-N-[(2S)-4-amino-2-hydroxybutanoyl]-2-deoxy-D-streptamine. Arbekacin is also described in JPS56-051499A and JPS58-134099A. In addition, arbekacin is also described in the Japanese Pharmacopoeia 18th Edition, Official Monographs. In the present specification, the concentration of arbekacin and/or a salt thereof is the concentration in terms of arbekacin, unless otherwise specified.

In the present specification, "hydroxypropyl methylcellulose" is a type of cellulose polymer and refers to a methyl and hydroxypropyl mixed ether of cellulose. Hydroxypropyl methylcellulose is also referred to as hypromellose. It is sometimes abbreviated as HPMC.

In the present specification, the "viscosity" of the aqueous liquid formulation is measured according to "2.1.3. Cone-Plate Rotational Viscometer (Cone-Plate Viscometer))" in "2. Method 2 Rotational Viscometer Method" in "2.53 Viscosity Determination" prescribed in the Japanese Pharmacopoeia 18th Edition, General Tests (25°C$\pm$0.1°C, preheating time: 0 s, rotation speed: 50 rpm, cone-plate rotational viscometer, cone rotor used: 1°34'xR24, measurement time: 90 s). Specifically, the viscosity of the aqueous liquid formulation is measured by using a TVE-25 type viscometer (model: TVE-25L) produced by Toki Sangyo Co., Ltd.

In the present specification, "viscosity stabilization" refers to suppressing the decrease in the viscosity of the aqueous liquid formulation over time. The "viscosity retention rate" is an index showing the degree of "viscosity stabilization," and a higher value indicates more stable viscosity. For example, the viscosity retention rate when stored at a predetermined temperature for a predetermined period is calculated according to the following formula: Viscosity retention rate (%) = viscosity (mPa·s) of aqueous liquid formulation at each storage time point/viscosity (mPa·s) of aqueous liquid formulation at the time of preparation $\times$ 100

## 2. Aqueous Liquid Formulation

The aqueous liquid formulation included in the present disclosure comprises arbekacin and/or a salt thereof, hydroxypropyl methylcellulose, and propylene glycol. In the present specification, the aqueous liquid formulation is also referred to as "the aqueous liquid formulation of the present disclosure."

Arbekacin is one of the aminoglycoside antibiotics. Arbekacin sulfate is used to treat pneumonia and sepsis caused by methicillin-resistant *Staphylococcus aureus* (MRSA) (PTL 1). In addition, arbekacin sulfate reportedly exhibits a broad range of antibacterial activity not only against gram-positive bacteria, including methicillin-resistant *Staphylococcus aureus,* but also against gram-negative bacteria (NPL 1).

Salts of arbekacin are not particularly limited as long as they are pharmaceutically acceptable. Examples include organic acid salts and inorganic acid salts. Examples of organic acid salts include tartrate, acetate, and the like. Examples of inorganic acid salts include sulfate, hydrochloride, and the like. Arbekacin or a salt thereof may be in the form of a solvate such as a hydrate. Among arbekacin or salts thereof, arbekacin sulfate is preferably used because it is commercially available as a pharmaceutical and its safety has been established.

In the aqueous liquid formulation of the present disclosure, either arbekacin or a salt thereof may be used alone, or they may be used in combination.

The total concentration of arbekacin and/or a salt thereof in the aqueous liquid formulation of the present disclosure can be, for example, about 0.05 to about 5.0 w/v% in terms of arbekacin. The upper or lower limit of this range may be, for example, about 0.1, about 0.2, about 0.3, about 0.4, about 0.5, about 0.6, about 0.7, about 0.8, about 0.9, about 1.0, about 1.5, about 2.0, about 2.5, about 3.0, about 3.5, about 4.0, or about 4.5 w/v%. For example, the range can be preferably about 0.1 to about 3.0 w/v%.

The methoxy group content of hydroxypropyl methylcellulose in the aqueous liquid formulation of the present disclosure is preferably, for example, about 15 mass% to about 32 mass%. The upper or lower limit of this range may be, for example, about 15.5, about 16, about 16.5, about 17, about 17.5, about 18, about 18.5, about 19, about 19.5, about 20, about 20.5, about 21, about 21.5, about 22, about 22.5, about 23, about 23.5, about 24, about 24.5, about 25, about 25.5, about 26, about 26.5, about 27, about 27.5, about 28, about 28.5, about 29, about 29.5, about 30, about 30.5, about 31, or about 31.5 mass%. The range may be, for example, about 16 mass% to 30 mass%.

The hydroxypropoxy group content of hydroxypropyl methylcellulose in the aqueous liquid formulation of the present disclosure is preferably, for example, about 3 mass% to about 35 mass%. The upper or lower limit of this range may be, for example, about 3.5, about 4, about 4.5, about 5, about 5.5, about 6, about 6.5, about 7, about 7.5, about 8, about 8.5, about 9, about 9.5, about 10, about 10.5, about 11, about 11.5, about 12, about 12.5, about 13, about 13.5, about 14, about 14.5, about 15, about 15.5, about 16, about 16.5, about 17, about 17.5, about 18, about 18.5, about 19, about 19.5, about 20, about 20.5, about 21, about 21.5, about 22, about 22.5, about 23, about 23.5, about 24, about 24.5, about 25, about 25.5, about 26, about 26.5, about 27, about 27.5, about 28, about 28.5, about 29, about 29.5, about 30, about 30.5, about 31, about 31.5, about 32, about 32.5, about 33, about 33.5, about 34, or about 34.5 mass%. The range may be, for example, about 4 mass% to 33 mass%.

The contents of methoxy and hydroxypropoxy groups are preferably combinations of the above ranges. Preferred are those having a methoxy group content of about 15 mass% to about 32 mass% and a hydroxypropoxy group content of about 3 mass% to about 35 mass%. In particular, more preferred are, for example, one having a methoxy group content of about 16.5 mass% to about 20.0 mass% and a hydroxypropoxy group content of about 23.0 mass% to about 32.0 mass%, one having a methoxy group content of about 19.0 mass% to about 24.0 mass% and a hydroxypropoxy group content of about 4.0 mass% to about 12.0 mass%, one having a methoxy group content of about 27.0 mass% to about 30.0 mass% and a hydroxypropoxy group content of about 4.0 mass% to about 7.5 mass%, and one having a methoxy group content of about 28.0 mass% to about 30.0 mass% and a hydroxypropoxy group content of about 7.0 mass% to about 12.0 mass%.

In the Japanese Pharmacopoeia 18th Edition, the substitution type of hydroxypropyl methylcellulose is defined by the contents of methoxy and hydroxypropoxy groups as follows. The substitution type 1828 refers to one in which the lower limit of the methoxy group content is about 16.5 mass%, the upper limit of the methoxy group content is about 20.0 mass%, the lower limit of the hydroxypropoxy group content is about 23.0 mass%, and the upper limit of the hydroxypropoxy group content is about 32.0 mass% (i.e., a methoxy group content of about 16.5 mass% to about 20.0 mass% and a hydroxypropoxy group content of about 23.0 mass% to about 32.0 mass%). The substitution type 2208 refers to one in which the lower limit of the methoxy group content is about 19.0 mass%, the upper limit of the methoxy group content is about 24.0 mass%, the lower limit of the hydroxypropoxy group content is about 4.0 mass%, and the upper limit of the hydroxypropoxy group content is about 12.0 mass% (i.e., a methoxy group content of about 19.0 mass% to about 24.0 mass% and a hydroxypropoxy group content of about 4.0 mass% to about 12.0 mass%). The substitution type 2906 refers to one in which the lower limit of the methoxy group content is about 27.0 mass%, the upper limit of the methoxy group content is about 30.0 mass%, the lower limit of the hydroxypropoxy group content is about 4.0 mass%, and the upper limit of the hydroxypropoxy group content is about 7.5 mass% (i.e., a methoxy group content of about 27.0 mass% to about 30.0 mass% and a hydroxypropoxy group content of about 4.0 mass% to about 7.5 mass%). The substitution type 2910 refers to one in which the lower limit of the methoxy group content is about 28.0 mass%, the upper limit of the methoxy group content is about 30.0 mass%, the lower limit of the hydroxypropoxy group content is about 7.0 mass%, and the upper limit of the hydroxypropoxy group content is about 12.0 mass% (i.e., a methoxy group content of about 28.0 mass% to about 30.0 mass% and a hydroxypropoxy group content of about 7.0 mass% to about 12.0 mass%).

The substitution type of hydroxypropyl methylcellulose in the aqueous liquid formulation of the present disclosure is not particularly limited. For example, the substitution type may be any of 1828, 2208, 2906, and 2910. The substitution type is preferably 2208 or 2910.

The molecular weight of hydroxypropyl methylcellulose is not particularly limited. For example, the weight average molecular weight is about 10,000 to about 500,000, preferably about 50,000 to about 500,000, and more preferably about 50,000 to about 300,000. The weight average molecular weight can be determined by gel permeation chromatography (GPC) using polystyrene as a standard substance.

The concentration of hydroxypropyl methylcellulose in the aqueous liquid formulation of the present disclosure can be, for example, about 0.2 to about 3.5 w/v%. The concentration of hydroxypropyl methylcellulose is preferably about 0.3 to about 2.0 w/v%, more preferably about 0.3 to about 1.5 w/v%, even more preferably about 0.8 to about 1.5 w/v%, and particularly preferably about 0.8 to about 1.4 w/v%.

The concentration of propylene glycol in the aqueous liquid formulation of the present disclosure can be, for example, about 0.1 to about 10 w/v%. The concentration of propylene glycol is preferably about 0.1 to about 5 w/v%, more preferably about 0.2 to about 4 w/v%, even more preferably about 0.5 to about 3 w/v%, and particularly preferably about 1.0 to about 2.5 w/v%.

In the aqueous liquid formulation of the present disclosure, the ratio of propylene glycol to arbekacin and/or a salt thereof is not particularly limited. For example, the mass ratio of the total content of arbekacin and/or a salt thereof in terms of arbekacin and the content of propylene glycol can be 1:about 0.02 to 1:about 200 parts by mass. For example, the mass ratio may be 1:about 0.05 to 1:about 100 parts by mass, 1:about 0.75 to 1:about 80 parts by mass, 1:about 0.1 to 1:about 60 parts by mass, 1:about 0.1 to 1:about 30 parts by mass, 1:about 0.1 to 1:about 10 parts by mass, 1:about 0.2 to 1:about 5 parts by mass, or 1:about 0.3 to 1:about 1 part by mass.

In the aqueous liquid formulation of the present disclosure, the ratio of propylene glycol to hydroxypropyl methylcellulose is not particularly limited. For example, the mass ratio of the content of hydroxypropyl methylcellulose and the content of propylene glycol can be 1:about 0.028 to 1:about 50 parts by mass. For example, the mass ratio may be 1:about 0.1 to 1:about 25 parts by mass, 1:about 0.2 to 1:about 20 parts by mass, 1:about 0.4 to 1:about 10 parts by mass, or 1:about 0.85 to 1:about 2.7 parts by mass.

In the aqueous liquid formulation of the present disclosure, the ratio of hydroxypropyl methylcellulose to arbekacin and/or a salt thereof is not particularly limited. For example, the mass ratio of the total content of arbekacin and/or a salt thereof in terms of arbekacin and the content of hydroxypropyl methylcellulose can be 1:about 0.04 to 1:about 70 parts by mass. The mass ratio may be, for example, 1:about 0.1 to 1:about 20 parts by mass, or 1:about 0.25 to 1:about 14 parts by mass.

In the aqueous liquid formulation of the present disclosure, the ratio of arbekacin and/or a salt thereof, hydroxypropyl methylcellulose, and propylene glycol is not particularly limited. For example, the mass ratio of the total content of arbekacin and/or a salt thereof in terms of arbekacin, the content of hydroxypropyl methylcellulose, and the content of propylene glycol can be 1:about 0.04:about 0.02 to 1:about 70:about 200 parts by mass. The mass ratio may be, for example, 1:about 0.1:about 0.1 to 1:about 50:about 100 parts by mass, or 1:about 0.5:about 0.5 to 1:about 25:about 50 parts by mass (i.e., may be, in terms of parts by mass, 1:about 0.04 to about 70:about 0.02 to about 200, or 1:about 0.5 to about 25:about 0.5 to about 50).

The aqueous liquid formulation of the present disclosure may contain, if necessary, additives such as buffers, isotonic agents, thickening agents, surfactants, antiseptics or preservatives, refreshing agents, stabilizers, and pH adjusters, as long as the effects of the aqueous liquid formulation of the present disclosure are not impaired.

Buffers are not particularly limited as long as they are pharmaceutically acceptable. Examples include borate buffers, citrate buffers, phosphate buffers, Tris buffers, tartrate buffers, acetate buffers, amino acid buffers, and the like. These buffers may be used singly or in combination of two or more.

Specific examples of borate buffers include boric acid and/or salts thereof. Boric acid is not particularly limited as long as it is pharmaceutically acceptable. Examples include orthoboric acid, metaboric acid, tetraboric acid, and the like. Preferred among these boric acids are orthoboric acid and tetraboric acid. These boric acids may be used singly or in combination of two or more. Salts of boric acid are not particularly limited as long as they are pharmaceutically acceptable. Examples include alkali metal salts, such as sodium salts and potassium salts; alkaline earth metal salts, such as calcium salts and magnesium salts; aluminum salts; organic amine salts, such as triethylamine, triethanolamine, morpholine, piperazine, and pyrrolidine; and the like. Boric acid and/or a salt thereof may be in the form of a hydrate, such as borax. As the borate buffer, one may be selected from boric acid and salts thereof and used alone, or two or more may be used in combination. Preferred among boric acid and salts thereof is at least one of boric acid and borax, and more preferred is at least one of orthoboric acid and borax.

Specific examples of citrate buffers include citric acid and/or salts thereof. Salts of citric acid are not particularly limited as long as they are pharmaceutically acceptable. Examples include alkali metal salts, such as sodium salts and potassium salts; alkaline earth metal salts, such as calcium salts and magnesium salts; and the like. The salt of citric acid may also be in the form of a solvate, such as a hydrate. As the citrate buffer, one may be selected from citric acid and salts thereof and used alone, or two or more may be used in combination. Preferred among citric acid and salts thereof are salts of citric acid, more preferred are alkali metal salts of citric acid, and particularly preferred is sodium citrate.

Specific examples of phosphate buffers include phosphoric acid and/or salts thereof. Salts of phosphoric acid are not particularly limited as long as they are pharmaceutically acceptable. Examples include dialkali metal hydrogen phosphates, such as disodium hydrogen phosphate and dipotassium hydrogen phosphate; alkali metal dihydrogen phosphates, such as sodium dihydrogen phosphate and potassium dihydrogen phosphate; trialkali metal phosphates, such as trisodium phosphate and tripotassium phosphate; and the like. The salt of phosphoric acid may also be in the form of a solvate, such as a hydrate. For example, in the case of disodium hydrogen phosphate, it may be in the form of a dodecahydrate, and in the form of sodium dihydrogen phosphate, it may be in the form of a dihydrate. As the phosphate buffer, one may be selected from phosphoric acid and salts thereof and used alone, or two or more may be used in combination. Preferred among phosphoric acid and salts thereof are phosphates, more preferred is at least one of dialkali metal hydrogen phosphates and alkali metal dihydrogen phosphates, and particularly preferred is at least one of disodium hydrogen phosphate and sodium dihydrogen phosphate.

Specific examples of Tris buffers include trometamol and/or salts thereof. Salts of trometamol are not particularly limited as long as they are pharmaceutically acceptable. Examples include organic acid salts, such as acetic acid salts; and inorganic acid salts, such as hydrochloride and sulfonate. As the Tris buffer, one may be selected from trometamol and salts thereof and used alone, or two or more may be used in combination. Preferred among trometamol and salts thereof is trometamol.

Specific examples of tartrate buffers include tartaric acid and/or salts thereof. Salts of tartaric acid are not particularly limited as long as they are pharmaceutically acceptable. Examples include alkali metal salts, such as sodium salts and potassium salts; alkaline earth metal salts, such as calcium salts and magnesium salts; and the like. The salt of tartaric acid may also be in the form of a solvate, such as a hydrate. As the tartrate buffer, one may be selected from tartaric acid and salts thereof and used alone, or two or more may be used in combination.

Specific examples of acetate buffers include acetic acid and/or salts thereof. Salts of acetic acid are not particularly limited as long as they are pharmaceutically acceptable. Examples include alkali metal salts, such as sodium salts and potassium salts; alkaline earth metal salts, such as calcium salts and magnesium salts; ammonium salts; and the like. The salt of acetic acid may also be in the form of a solvate, such as a hydrate. As the acetate buffer, one may be selected from acetic acid and salts thereof and used alone, or two or more may be used in combination.

Specific examples of amino acid buffers include acidic amino acid and/or salts thereof. Specific examples of acidic amino acid include aspartic acid and glutamic acid. Salts of acidic amino acid are not particularly limited as long as they are pharmaceutically acceptable. Examples include alkali metal salts, such as sodium salts and potassium salts. As the amino acid buffer, one may be selected from acidic amino acid and salts thereof and used alone, or two or more may be used in combination.

The concentration of the buffer in the aqueous liquid formulation of the present disclosure may be appropriately set within a range that can impart the desired buffering capacity to the aqueous liquid formulation. The concentration of the buffer can be, for example, about 0.01 to about 3.0 w/v%.

Isotonic agents are not particularly limited as long as the effects of the aqueous liquid formulation of the present disclosure are not impaired and they are pharmaceutically acceptable. Examples include polyhydric alcohols other than propylene glycol, such as glycerin, butylene glycol, polyethylene glycol, and mannitol; metal salts, such as sodium chloride, potassium chloride, calcium chloride, magnesium chloride, sodium acetate, potassium acetate, sodium hydrogen sulfite, sodium hydrogen carbonate, sodium carbonate, disodium hydrogen phosphate, and sodium dihydrogen phosphate; and the like. These isotonic agents may be used singly or in combination of two or more.

Thickening agents are not particularly limited as long as the effects of the aqueous liquid formulation of the present disclosure are not impaired and they are pharmaceutically acceptable. Examples include carboxyvinyl polymers, polyvinylpyrrolidone, polyacrylic acid, polyvinyl alcohol, xanthan gum, sodium chondroitin sulfate, sodium hyaluronate, celluloses other than hydroxypropyl methylcellulose, and other water-soluble polymers. Specific examples of celluloses herein include hydroxyethyl cellulose, methyl cellulose, hydroxypropyl cellulose, sodium carboxymethyl cellulose, and the like. These thickening agents may be used singly or in combination of two or more.

Surfactants are not particularly limited as long as they are pharmaceutically acceptable. Examples include nonionic surfactants, such as tyloxapol, polyoxyethylene hydrogenated castor oil, polyoxyethylene-polyoxypropylene block copolymers, polyoxyethylene sorbitan fatty acid esters, and octoxynol; amphoteric surfactants, such as alkyldiaminoethylglycine and lauryldimethylaminoacetate betaine; anionic surfactants, such as alkyl sulfates, N-acyl taurine salts, polyoxyethylene alkyl ether phosphates, and polyoxyethylene alkyl ether sulfates; cationic surfactants, such as alkylpyridinium salts and alkylamine salts; and the like. These surfactants may be used singly or in combination of two or more.

Antiseptics or preservatives are not particularly limited as long as they are pharmaceutically acceptable. Examples include sorbic acid or salts thereof, benzoic acid or salts thereof, methyl parahydroxybenzoate, ethyl parahydroxybenzoate, propyl parahydroxybenzoate, chlorobutanol, benzalkonium chloride, chlorhexidine hydrochloride, chlorhexidine gluconate, chlorhexidine acetate, dehydroacetic acid or salts thereof, benzethonium chloride, benzyl alcohol, zinc chloride, zinc sulfate, silver nitrate, polyhexanide, alkyldiaminoethylglycine hydrochloride, parachlorometaxylenol, chlorocresol, phenethyl alcohol, polidronium chloride, thimerosal, dibutylhydroxytoluene, and the like. These antiseptics or preservatives may be used singly or in combination of two or more.

Refreshing agents are not particularly limited as long as they are pharmaceutically acceptable. Examples include l-menthol, borneol, camphor, eucalyptus oil, and the like. These refreshing agents may be used singly or in combination of two or more.

Stabilizers are not particularly limited as long as they are pharmaceutically acceptable. Examples include chelating agents, such as edetic acid, citric acid, succinic acid, ascorbic acid, trihydroxymethylaminomethane, nitrilotriacetic acid, 1-hydroxyethane-1,1-diphosphonic acid, polyphosphoric acid, metaphosphoric acid, hexametaphosphoric acid, and salts thereof; sodium thiosulfate, sulfite, monoethanolamine, cyclodextrin, dextran, ascorbic acid, taurine, tocopherol, dibutylhydroxytoluene, and the like. The form of salt is not particularly limited as long as it is pharmaceutically acceptable. Examples include alkali metal salts, such as sodium salts and potassium salts. These stabilizers may be used singly or in combination of two or more.

pH adjusters are not particularly limited as long as they are pharmaceutically acceptable. Examples include acids, such as hydrochloric acid, acetic acid, boric acid, aminoethylsulfonic acid, and epsilon-aminocaproic acid; and alkalis, such as sodium hydroxide, potassium hydroxide, borax, triethanolamine, monoethanolamine, sodium hydrogencarbonate, and sodium carbonate. These pH adjusters may be used singly or in combination of two or more.

The concentration of these additives may be appropriately set depending on the type of additive used and the characteristics to be imparted to the aqueous liquid formulation.

The pH of the aqueous liquid formulation of the present disclosure is not particularly limited as long as it is pharmaceutically acceptable. For example, the pH is about 5.0 to about 8.0. For example, the pH may be about 5.4 to about 7.5, about 5.4 to about 7.0, or about 5.4 to about 6.0.

The viscosity of the aqueous liquid formulation of the present disclosure is not particularly limited. For example, the viscosity immediately after preparation can be about 5.0 to about 100 mPa·s. For example, the viscosity may be about 5 to about 50 mPa·s, about 10 to about 35 mPa·s, or about 20 to about 35 mPa·s.

The osmotic pressure of the aqueous liquid formulation is the value measured according to the method specified in the Japanese Pharmacopoeia 18th Edition, "General Tests," "2.47 Osmotic Pressure Measurement Method (Osmolarity Determination)." The osmotic pressure of the aqueous liquid formulation of the present disclosure is not particularly limited as long as it is applicable to the intended use. For example, when applied to the ocular mucosa, the osmotic pressure is about 243 to about 350 mOsm/kg.

The osmotic pressure ratio of the aqueous liquid formulation refers to the ratio of the osmotic pressure of the aqueous liquid formulation to that of physiological saline (0.9 w/v% sodium chloride aqueous solution). The osmotic pressure ratio of the aqueous liquid formulation of the present disclosure is not particularly limited as long as it is applicable to the intended use. For example, when applied to the ocular mucosa, the osmotic pressure ratio is about 0.85 to about 1.15. In terms of relieving eye irritation, the osmotic pressure ratio is preferably about 0.8 to about 1.1, and more preferably about 1.0.

The formulation form of the aqueous liquid formulation of the present disclosure is not particularly limited, and may be in the form of an aqueous solution, a suspension, an emulsion, or the like. A preferred form is an aqueous solution.

The aqueous liquid formulation of the present disclosure may be prepared according to a known preparation method appropriate for the dosage form, for example, using the method described in the Japanese Pharmacopoeia 18th Edition.

Specifically, for example, the method for producing the aqueous liquid formulation of the present disclosure comprises the step of mixing arbekacin and/or a salt thereof, hydroxypropyl methylcellulose, and propylene glycol in a pharmaceutically acceptable aqueous medium. The "pharmaceutically acceptable aqueous medium" means an aqueous medium that is pharmaceutically acceptable, such as purified water. In the mixing step, the order of mixing each component is not particularly limited, and the components may be mixed sequentially in any order or may be mixed simultaneously.

The aqueous liquid formulation of the present disclosure is, for example, prepared into pharmaceutical compositions for various applications, such as ophthalmology, dentistry, otolaryngology, and dermatology, and are used as topical administration formulations. The aqueous liquid formulation of the present disclosure can be used, for example, as a composition for ophthalmology, dentistry, otolaryngology, dermatology, or the like. Preferred is an ophthalmic composition.

Specific examples of the ophthalmic composition include eye drops, eyewashes, contact lens solutions, injections, and the like. Preferred among these are eye drops.

Arbekacin and/or a salt thereof contained in the aqueous liquid formulation of the present disclosure has antibacterial effects against gram-positive and gram-negative bacteria. Therefore, the aqueous liquid formulation of the present disclosure can be suitably used for treating, for example, bacterial external ocular infections and bacterial keratoconjunctivitis (bacterial conjunctivitis and/or bacterial keratitis), and can be more suitably used for treating bacterial conjunctivitis.

The causative bacteria of bacterial external ocular infections include gram-positive, gram-negative bacteria, and the like. Examples of gram-positive bacteria that cause bacterial conjunctivitis include *Staphylococcus aureus* (e.g., methicillin-resistant *Staphylococcus aureus*), pneumococcus, *Corynebacterium*, and the like. Examples of gram-negative bacteria that cause bacterial conjunctivitis include *Haemophilus influenzae, Moraxella, Neisseria gonorrhoeae*, and the like (Noriko INADA, Journal of Clinical Ophthalmology, Vol. 75, No. 11, 2021).

Examples of the causative bacteria of bacterial keratitis include pneumococcus, *Staphylococcus, Pseudomonas aeruginosa, Moraxella, Serratia, Streptococcus, Neisseria gonorrhoeae*, anaerobic bacteria, atypical mycobacteria, and the like (Guidelines for the clinical management of infectious keratitis (2nd edition), Japanese Ophthalmological Society, 2013).

A single bacterium or a combination of two or more bacteria may cause bacterial external ocular infections and bacterial keratoconjunctivitis.

The causative bacteria of bacterial external ocular infections (more specifically bacterial keratoconjunctivitis) are preferably *Staphylococcus* (e.g., methicillin-resistant *Staphylococcus aureus*), *Corynebacterium, Pseudomonas aeruginosa, Haemophilus influenzae*, pneumococcus, *Moraxella, Neisseria gonorrhoeae, Serratia*, or *Streptococcus*; more preferably *Staphylococcus* (e.g., methicillin-resistant *Staphylococcus aureus*), *Corynebacterium, Pseudomonas aeruginosa, Haemophilus influenzae*, or pneumococcus; even more preferably *Staphylococcus* (e.g., methicillin-resistant *Staphylococcus aureus*), and particularly preferably methicillin-resistant *Staphylococcus aureus*.

Examples of the administration subject of the aqueous liquid formulation of the present disclosure include humans and non-human mammals (e.g., rats, mice, rabbits, cows, pigs, dogs, cats, sheep, and monkeys). Examples of humans to be administered include patients with bacterial keratoconjunctivitis or humans suspected of bacterial keratoconjunctivitis, humans infected with the bacteria mentioned above, and the like. More specifically, preferred are humans infected with or suspected of being infected with *Staphylococcus* (e.g., methicillin-resistant *Staphylococcus aureus), Corynebacterium, Pseudomonas aeruginosa, Haemophilus influenzae,* pneumococcus, *Moraxella, Neisseria gonorrhoeae, Serratia,* or *Streptococcus*; more preferred are humans infected with or suspected of being infected with *Staphylococcus* (e.g., methicillin-resistant *Staphylococcus aureus), Corynebacterium, Pseudomonas aeruginosa, Haemophilus influenzae,* or pneumococcus; even more preferred are humans infected with or suspected of being infected with *Staphylococcus* (e.g., methicillin-resistant *Staphylococcus aureus*); and particularly preferred are humans infected with or suspected of being infected with methicillin-resistant *Staphylococcus aureus.*

Other examples of humans to be administered include patients with dry eye or humans suspected of dry eye, patients with bacterial external ocular infections (more specifically bacterial keratoconjunctivitis) with dry eye or humans suspected of bacterial external ocular infections (more specifically bacterial keratoconjunctivitis) with dry eye, and the like.

The dosage (intake) of the aqueous liquid formulation of the present disclosure is not particularly limited, and is determined depending on the age, weight, sex, severity of symptoms, administration method, etc. of the subject to be administered. For example, the dosage of arbekacin can be about 0.004 to about 1.5 mg/kg of body weight per day.

When the aqueous liquid formulation of the present disclosure is used as an eye drop, a few drops (e.g., 1 to 3 drops) may be instilled into the eye one or more times (e.g., 2 to 8 times) per day. In one embodiment of the aqueous liquid formulation of the present disclosure, one drop is instilled into the eye twice a day.

The present disclosure also includes a method for stabilizing the viscosity of an aqueous liquid formulation, comprising preparing an aqueous liquid formulation comprising arbekacin and/or a salt thereof, hydroxypropyl methylcellulose, and propylene glycol. All of the above descriptions apply to the aqueous liquid formulation and its components.

In the present specification, the term "comprising" includes "consisting essentially of" and "consisting of." Further, the present disclosure encompasses any combination of the configuration elements described in the present specification.

In addition, the various characteristics (properties, structures, functions, etc.) described in each embodiment of the present disclosure described above may be combined in any way in specifying the subjects encompassed by the present disclosure. In other words, the present disclosure encompasses all the subjects comprising all combinations of the combinable characteristics described in the present specification.

Examples

The contents of the present disclosure will be described in detail using the following Experimental Examples and Examples. However, the present disclosure is not limited thereto. In the following, the experiments were carried out under atmospheric pressure and room temperature conditions unless otherwise specified. Further, "%" means "w/v%" unless otherwise specified. The amount of each component listed in each table is also in w/v % unless otherwise specified.

Test Example 1

An aqueous liquid formulation was prepared by mixing components to obtain the composition shown in Table 1A.

Table 1A

| Component name | Amount mixed (%) |
|---|---|
| Arbekacin (ARB) | See Table 1B |
| Propylene glycol (PG) | See Table 1B |
| Hypromellose 2208 (90SH-100SR, HPMC) | 1.4 |
| Hydrochloric acid | Appropriate amount |
| Sodium hydroxide | Appropriate amount |
| Purified water | Appropriate amount |
| pH | 5.5 |

The viscosity of the prepared aqueous liquid formulation was measured according to the following conditions and recorded as the viscosity at the time of preparation. In addition, 5 mL of the prepared aqueous liquid formulation was placed in a colorless glass ampoule (volume: 5 mL), which was then stored at 50°C for 2, 4, and 8 weeks, after which the viscosity of the aqueous liquid formulation was measured.

Viscosity measurement conditions

Measuring device: TVE-25 type viscometer (Toki Sangyo Co., Ltd.)

Rotor: 1°34'xR24

Sample volume: 1.1 mL

Preheating time: 0 s

Measurement time: 90 s

Measurement temperature: 25°C±0.1°C

Rotation speed: 50 rpm

The viscosity retention rate at each storage time point was calculated using the following formula. The measurement results of the viscosity and viscosity retention rate are shown in Table 1B.

Viscosity retention rate (%) = viscosity (mPa·s) of aqueous liquid formulation at each storage time point/viscosity (mPa·s) of aqueous liquid formulation at the time of preparation (at the start) × 100

Table 1B

| | | Amount mixed (%) | | At the start | 50°C/2 weeks | | 50°C/4 weeks | | 50°C/8 weeks | |
|---|---|---|---|---|---|---|---|---|---|---|
| | Formulation No. | ARB | PG | Viscosity (mPa·s) | Viscosity (mPa·s) | Viscosity retention rate(%) | Viscosity (mPa·s) | Viscosity retention rate(%) | Viscosity (mPa·s) | Viscosity retention rate (%) |
| Comp. Ex. 1 | A01 | 0 | 0 | 29.72 | 27.96 | 94.1 | 27.78 | 93.5 | 26.40 | 88.8 |
| Comp. Ex. 2 | C01 | 0.1 | 0 | 27.56 | 24.69 | 89.6 | 23.23 | 84.3 | 19.91 | 72.2 |
| Ex. 1 | C03 | 0.1 | 1 | 30.22 | 28.59 | 94.6 | 28.74 | 95.1 | 26.69 | 88.3 |
| Ex. 2 | C05 | 0.1 | 3 | 31.86 | 30.65 | 96.2 | 31.10 | 97.6 | 29.86 | 93.7 |
| Comp. Ex. 3 | C13 | 1 | 0 | 31.28 | 26.55 | 84.9 | 24.23 | 77.5 | 18.10 | 57.9 |
| Ex. 3 | C15 | 1 | 1 | 32.23 | 29.37 | 91.1 | 27.98 | 86.8 | 23.69 | 73.5 |
| Ex. 4 | C17 | 1 | 3 | 33.00 | 31.18 | 94.5 | 30.31 | 91.8 | 27.65 | 83.8 |

Fig. 1 shows the viscosity retention rate at 50°C after two weeks. In Fig. 1, A01 is an aqueous liquid formulation not containing arbekacin and propylene glycol.

As shown in Comparative Examples 2 and 3, when an aqueous liquid formulation containing arbekacin and hypromellose was stored at 50°C, the viscosity retention rate decreased in proportion to the concentration of arbekacin. As shown in Examples 1 to 4, when propylene glycol was further added to an aqueous liquid formulation containing arbekacin and hypromellose, the viscosity retention rate increased in proportion to the concentration of propylene glycol. It was confirmed that the use of propylene glycol suppressed the decrease in viscosity and made it possible to maintain a practical viscosity.

Furthermore, an aqueous liquid formulation was prepared by mixing components to obtain the composition shown in Table 2A.

Table 2A

| Component name | Amount mixed (%) |
|---|---|
| Arbekacin (ARB) | See Table 2B |
| Propylene glycol (PG) | See Table 2B |
| Hypromellose 2208 (90SH-100SR, HPMC) | 1.4 |
| Hydrochloric acid | Appropriate amount |
| Sodium hydroxide | Appropriate amount |
| Purified water | Appropriate amount |
| pH | 5.5 |

The viscosity of the prepared aqueous liquid formulation was measured according to the following conditions and recorded as the viscosity at the time of preparation. In addition, 5 mL of the prepared aqueous liquid formulation was placed in a colorless glass ampoule (volume: 5 mL), which was then stored at 50°C for 8 weeks, after which the viscosity of the aqueous liquid formulation was measured in the same manner as described above, and the viscosity retention rate (%) was calculated using the above formula. The results are shown in Table 2B.

Table 2B

| | Amount mixed (%) | | At the start | 50°C/8 weeks | |
|---|---|---|---|---|---|
| | ARB | PG | Viscosity (mPa·s) | Viscosity (mPa·s) | Viscosity retention rate (%) |
| Reference Example A | 0 | 1 | 27.58 | 26.37 | 95.6 |
| Reference Example B | 0 | 3 | 28.06 | 27.05 | 96.4 |
| Comparative Example A | 3 | 0 | 28.58 | 18.54 | 64.9 |
| Example A | 3 | 1 | 30.77 | 21.67 | 70.4 |
| Example B | 3 | 3 | 33.09 | 25.84 | 78.1 |

In the aqueous liquid formulations containing a relatively high concentration (3 w/v%) of arbekacin, when propylene glycol was further added to the aqueous liquid formulations containing arbekacin and hypromellose, the viscosity retention rate increased in proportion to the concentration of propylene glycol, as with the above examination results.

Test Example 2

Aqueous liquid formulations having the compositions shown in Tables 3 to 6 are prepared and evaluated in the same manner as in Test Example 1. The compositions of Examples 12 and 14 and Comparative Example 8 are the same as those of Examples A and B and Comparative Example A, respectively.

Table 3

| Component name | Amount mixed (%) | | | | | | |
|---|---|---|---|---|---|---|---|
| | Comparative Example 4 | Comparative Example 5 | Example 5 | Comparative Example 6 | Example 6 | Comparative Example 7 | Example 7 |
| Arbekacin | 0 | 0.1 | 0.1 | 1 | 1 | 3 | 3 |
| Propylene glycol | 0 | 0 | 1.5 | 0 | 1.5 | 0 | 1 |
| Hypromellose (HPMC) | 1 | 1 | 1 | 1 | 1 | 1 | 1 |
| Hydrochloric acid | Appropriate amount | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount | Appropriate amount |
| Purified water | Appropriate amount | Appropriate amount | Appropriate amount | Appropriate amount | Appropriate amount | Appropriate amount | Appropriate amount |
| pH | 5.5 | 5.5 | 5.5 | 5.5 | 5.5 | 5.5 | 5.5 |

Table 4

| Component name | Amount mixed (%) | | | | | | |
|---|---|---|---|---|---|---|---|
| | Example 8 | Example 9 | Example 10 | Example 11 | Comparative Example 8 | Example 12 | Example 13 |
| Arbekacin | 3 | 3 | 0.1 | 1 | 3 | 3 | 3 |
| Propylene glycol | 1.5 | 3 | 1.5 | 1.5 | 0 | 1 | 1.5 |
| Hypromellose (HPMC) | 1 | 1 | 1.4 | 1.4 | 1.4 | 1.4 | 1.4 |
| Hydrochloric acid | Appropriate amount | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount | Appropriate amount |
| Purified water | Appropriate amount | Appropriate amount | Appropriate amount | Appropriate amount | Appropriate amount | Appropriate amount | Appropriate amount |
| pH | 5.5 | 5.5 | 5.5 | 5.5 | 5.5 | 5.5 | 5.5 |

Table 5

| Component name | Amount mixed (%) | | | | | | |
|---|---|---|---|---|---|---|---|
| | Example 14 | Comparative Example 9 | Comparative Example 10 | Example 15 | Example 16 | Example 17 | Comparative Example 11 |
| Arbekacin | 3 | 0 | 0.1 | 0.1 | 0.1 | 0.1 | 1 |
| Propylene glycol | 3 | 0 | 0 | 1 | 1.5 | 3 | 0 |
| Hypromellose (HPMC) | 1.4 | 2 | 2 | 2 | 2 | 2 | 2 |
| Hydrochloric acid | Appropriate amount | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount | Appropriate amount |
| Purified water | Appropriate amount | Appropriate amount | Appropriate amount | Appropriate amount | Appropriate amount | Appropriate amount | Appropriate amount |
| pH | 5.5 | 5.5 | 5.5 | 5.5 | 5.5 | 5.5 | 5.5 |

Table 6

| Component name | Amount mixed (%) | | | | | | |
|---|---|---|---|---|---|---|---|
| | Example 18 | Example 19 | Example 20 | Comparative Example 12 | Example 21 | Example 22 | Example 23 |
| Arbekacin | 1 | 1 | 1 | 3 | 3 | 3 | 3 |
| Propylene glycol | 1 | 1.5 | 3 | 0 | 1 | 1.5 | 3 |
| Hypromellose (HPMC) | 2 | 2 | 2 | 2 | 2 | 2 | 2 |
| Hydrochloric acid | Appropriate amount | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount | Appropriate amount |
| Purified water | Appropriate amount | Appropriate amount | Appropriate amount | Appropriate amount | Appropriate amount | Appropriate amount | Appropriate amount |
| pH | 5.5 | 5.5 | 5.5 | 5.5 | 5.5 | 5.5 | 5.5 |

**Claims**

1. An aqueous liquid formulation comprising arbekacin and/or a salt thereof, hydroxypropyl methylcellulose, and propylene glycol.

2. The aqueous liquid formulation according to claim 1, wherein the total concentration of arbekacin and/or a salt thereof is about 0.05 w/v% to about 5.0 w/v% in terms of arbekacin.

3. The aqueous liquid formulation according to claim 1, wherein the concentration of hydroxypropyl methylcellulose is about 0.2 w/v% to about 3.5 w/v%.

4. The aqueous liquid formulation according to claim 1, wherein the concentration of propylene glycol is about 0.1 w/v% to about 10 w/v%.

5. The aqueous liquid formulation according to claim 1, wherein the total concentration of arbekacin and/or a salt thereof is about 0.05 w/v% to about 5.0 w/v% in terms of arbekacin, and the concentration of hydroxypropyl methylcellulose is about 0.2 w/v% to about 3.5 w/v%.

6. The aqueous liquid formulation according to claim 1, wherein the total concentration of arbekacin and/or a salt thereof is about 0.05 w/v% to about 5.0 w/v% in terms of arbekacin, and the concentration of propylene glycol is about 0.1 w/v% to about 10 w/v%.

7. The aqueous liquid formulation according to claim 1, wherein the concentration of hydroxypropyl methylcellulose is about 0.2 w/v% to about 3.5 w/v%, and the concentration of propylene glycol is about 0.1 w/v% to about 10 w/v%.

8. The aqueous liquid formulation according to claim 1, wherein the total concentration of arbekacin and/or a salt thereof is about 0.05 w/v% to about 5.0 w/v% in terms of arbekacin, the concentration of hydroxypropyl methylcellulose is about 0.2 w/v% to about 3.5 w/v%, and the concentration of propylene glycol is about 0.1 w/v% to about 10 w/v%.

9. The aqueous liquid formulation according to any one of claims 1 to 8, wherein the mass ratio of the total content of arbekacin and/or a salt thereof in terms of arbekacin and the content of propylene glycol is 1:about 0.02 to 1:about 200.

10. The aqueous liquid formulation according to any one of claims 1 to 8, wherein the mass ratio of the content of hydroxypropyl methylcellulose and the content of propylene glycol is 1:about 0.028 to 1:about 50.

11. The aqueous liquid formulation according to any one of claims 1 to 8, wherein the mass ratio of the total content of arbekacin and/or a salt thereof in terms of arbekacin and the content of hydroxypropyl methylcellulose is 1:about 0.04 to 1:about 70.

12. The aqueous liquid formulation according to any one of claims 1 to 8, wherein the mass ratio of the total content of arbekacin and/or a salt thereof in terms of arbekacin, the content of hydroxypropyl methylcellulose, and the content of propylene glycol is 1:about 0.04:about 0.02 to 1:about 70:about 200.

13. The aqueous liquid formulation according to any one of claims 1 to 8, which is an eye drop.

14. A method for stabilizing the viscosity of an aqueous liquid formulation, comprising preparing an aqueous liquid formulation comprising arbekacin and/or a salt thereof, hydroxypropyl methylcellulose, and propylene glycol.

Fig. 1

## INTERNATIONAL SEARCH REPORT

| International application No. |
|---|
| **PCT/JP2024/026008** |

### A. CLASSIFICATION OF SUBJECT MATTER

*A61K 31/7008*(2006.01)i; *A61K 9/08*(2006.01)i; *A61K 47/10*(2017.01)i; *A61K 47/38*(2006.01)i; *A61P 31/04*(2006.01)i
FI:   A61K31/7008; A61K9/08; A61K47/38; A61K47/10; A61P31/04

According to International Patent Classification (IPC) or to both national classification and IPC

### B. FIELDS SEARCHED

Minimum documentation searched (classification system followed by classification symbols)

A61K31/7008; A61K9/08; A61K47/10; A61K47/38; A61P31/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2018/003796 A1 (LION CORPORATION) 04 January 2018 (2018-01-04) <br> claims 1, 3, 5, paragraphs [0004], [0012], [0026]-[0028], [0035], [0044], examples 11, 13, 24, tables 2-4 | 1-14 |
| Y | WO 2018/066651 A1 (LION CORPORATION) 12 April 2018 (2018-04-12) <br> claims 1, 3, 5, paragraphs [0004], [0012], [0031]-[0033], [0040], [0049], examples 1-2, 2-2, 3-4, 4-1, 4-3, 4-4, 20-1, 24-1, 24-2, tables 1, 4, 6, 8, 19, 22 | 1-14 |
| Y | WO 2005/046700 A1 (SENJU PHARMACEUTICAL CO., LTD.) 26 May 2005 (2005-05-26) <br> claims 1, 8, paragraph [0009] | 1-14 |
| A | CN 106420811 A (CHANGZHOU RUNNUO BIOTECHNOLOGY CO., LTD.) 22 February 2017 (2017-02-22) <br> claim 1, paragraphs [0014], [0019], [0021] | 1-14 |
| A | CN 106474149 A (CHANGZHOU RUNNUO BIOLOGICAL TECHNOLOGY CO., LTD.) 08 March 2017 (2017-03-08) <br> claim 1, paragraphs [0015], [0020], [0022] | 1-14 |

☐ Further documents are listed in the continuation of Box C.      ☑ See patent family annex.

| | |
|---|---|
| \* Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **13 September 2024** | **01 October 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

## INTERNATIONAL SEARCH REPORT
### Information on patent family members

International application No.

**PCT/JP2024/026008**

| Patent document cited in search report | | | Publication date (day/month/year) | Patent family member(s) | | | Publication date (day/month/year) |
|---|---|---|---|---|---|---|---|
| WO | 2018/003796 | A1 | 04 January 2018 | KR 10-2019-0025810 | A | | |
| | | | | TW 201801719 | A | | |
| WO | 2018/066651 | A1 | 12 April 2018 | KR 10-2019-0065237 | A | | |
| | | | | TW 201813669 | A | | |
| WO | 2005/046700 | A1 | 26 May 2005 | US 2007/0082857 | A1 | | |
| | | | | claims 1, 8, paragraph [0020] | | | |
| | | | | EP 1683526 | A1 | | |
| | | | | KR 10-2006-0120084 | A | | |
| | | | | CN 1882349 | A | | |
| CN | 106420811 | A | 22 February 2017 | (Family: none) | | | |
| CN | 106474149 | A | 08 March 2017 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9511143 B2
- JP 7224390 B
- JP S56051499 A
- JP S58134099 A

### Non-patent literature cited in the description

- *Clin Pharmacol.*, 26 September 2014, vol. 6, 139-48
- **NORIKO INADA**. *Journal of Clinical Ophthalmology*, 2021, vol. 75 (11)
- Guidelines for the clinical management of infectious keratitis. Japanese Ophthalmological Society. 2013